# EUROPEAN PATENT APPLICATION

(11) **EP 0 966 970 A1**
(43) Date of publication of application: **29.12.1999**
(21) Application number: 98950317.2
(22) Date of filing: 21.10.1998
(51) Int. Cl.: A61K 39/00, G01N 33/53, C07H 15/10, A61K 35/12

(54) **ANTIGEN SPECIFIC TO MENIERE'S DISEASE AND MEDICAL USE THEREOF**

(30) Priority: 07.11.1997 JP 32203897
(71) Applicant: OTSUKA PHARMACEUTICAL CO., LTD., Chiyoda-ku, Tokyo 101-0048 (JP)
(72) Inventor: IKEDA, Atsuko, Soka-shi, Saitama 340-0052 (JP); KOMATSUZAKI, Atsushi, Chiba-shi, Chiba 260-0004 (JP); HANDA, Shizuo, Tokyo 151-0064 (JP); TAKI, Takao, Itano-gun, Tokushima 771-0204 (JP)
(74) Representative: Hartz, Nikolai, Dr.
(86) International application number: JP9804758
(87) International publication number: WO9924064

(57) **Abstract**

An antigen specific to Ménière's disease and comprising a ganglioside having the sequence represented by NeuAcα2-3Galβ1-4GlcNAcβ1-3Galβ1-4GlcNAcβ1-3Galβ1-4Glcβ1-1Cer; a reagent kit containing the above specific antigen and used for detecting an antibody peculiar to Ménière's disease; an immunoassay for detecting an antibody peculiar to Ménière's disease by using the specific antigen; a reagent kit containing the above specific antigen and used for selectively isolating or removing an antibody peculiar to Ménière's disease; and a method for isolating or removing the above antibody.

## Description

### TECHNICAL FIELD

The present invention relates to a new use of a specific ganglioside originating from the acoustic nerve system. The ganglioside selectively reacts with an antibody which is possessed by a patient suffering from Ménière's disease and is unique to the disease. Therefore, the ganglioside can be advantageously used for diagnosis of and therapy for Ménière's disease, whose onset mechanism is considered to involve an autoantibody or an immune complex. Further, the present invention relates to a diagnostic method for Ménière's disease, a detecting reagent therefor, and a method for isolating or removing an antibody unique to the disease.

### BACKGROUND ART

Ménière's disease is a disease of the inner ear marked by recurrent attacks of vertigoes involving a labyrinthine syndrome (tinnitus and hearing disorder). The pathological profile of the disease has been clarified to involve endolymphatic hydrops in which endolymphatic space of the inner ear increases and endolymphatic fluid accumulates therein. Hitherto, the cause of the onset of the hydrops is suggested to be a disturbance in the autonomic nervous system. allergy, focal infection, virus, stress, metabolic disorder, or disendocriasis. However, the onset factor which explains the hydrops has not yet been elucidated.

Therefore, conventionally, Ménière's disease has been diagnosed by identifying the pathological profiles unique to the disease, including accumulation of lymph fluid in the inner ear, by use of an electrocochleogram, a glycerol test, or a furosemide test, but not by the onset factors. Therapies therefor include administration of osmotic diuretic agents for decreasing the endolymuphatic hydrops and a variety of surgical methods (endolymphatic-sac-decompression operation and inner-ear-destruction operation). However, their effects are limited only to alleviation or elimination of the symptoms.

Therefore, there remains need for early clarification of the onset factors of the disease, as well as establishment of fundamental diagnosis and therapeutic methods for the disease based on the onset factors.

Objects of the present invention include elucidation of the onset factors of Ménière's disease, and provision of a diagnostic method and a therapeutic method for the disease based on the onset factors. Particularly, the present invention provides an antigen specifically reacting with an autoantibody or an immune complex regarded as an onset factor of the disease, and also provides an immunological diagnostic method for Ménière's disease by use of the antigen, as well as a drug and a therapeutic method for treating the disease.

### DISCLOSURE OF THE INVENTION

The present inventors have studied the relationship between a ganglioside originating from the inner ear and Ménière's disease so as to clarify the onset factors of the disease and as a result have found that an autoantibody (an antiganglioside antibody) selectively reacting with ganglioside from the peripheral nervous system, which ganglioside resembles an internal acoustic ganglioside (Maguchi S. *et al*. (1991) Auris-Nasus-Larynx (Tokyo) 18, 1.); particularly, with a certain ganglioside which is obtained from acoustic neurinoma generating in the acoustic nerve, is present specifically in serum of a patient suffering Ménière's disease.

In recent years, in addition to the above-mentioned disturbance in the autonomic nervous system and allergy,autoimmunity has been newly discussed as a likely candidate onset factor of Ménière's disease. The autoimmunity theory is based on the following clinical facts: the concentration of an immune complex in blood increases with the onset of Ménière's disease; steroids provide satisfactory therapeutic results; and that Ménière's disease is accompanied by other autoimmunologic diseases. The theory is also based on the observation of endolymphatic hydrops in animals immunized with inner ear tissue or collagen serving as an antigen (Terayama Y, *at al*. (1964) Acta Otolaryngol 58. 49.). Therefore, the above-described findings in the present invention are considered to provide support for the autoimmunity theory.

The present invention provides an antigen specifically reacting with an antibody unique to Ménière's disease, which is newly regarded as an onset factor of the disease; particularly, a Ménière's-disease-specific antigen comprising a ganglioside having the following sequence: NeuAcα2-3Galβ1-4GlcNAcβ1-3Galβ1-4GlcNAcβ1-3Galβ1-4Glcβ1-1Cer.

The present invention also provides a reagent kit for detecting an antibody unique to Ménière's disease, characterized by comprising the above-described antigen specific to the disease.

The present invention further provides an immunoassay for detecting an antibody unique to Ménière's disease, characterized by causing the Ménière's-disease-specific antigen to react with biological components and detecting a produced antigen-antibody complex comprising the specific antigen.

The present invention still further provides a reagent kit for selective isolation or removal of an antibody unique to Ménière's disease, comprising the above-described antigen specific to the disease.

The present invention yet further provides a method for isolating or removing an antibody unique to Ménière's disease, characterized by bringing the above-described antigen specific to Ménière's disease into contact with biological components having an antibody unique to the disease.

The present invention also provides a diagnostic method for Ménière's disease, characterized by causing the specific antigen described in claim 1 to react with biological components and detecting a produced antigen-antibody complex comprising the Ménière's-disease-specific antigen.

The present invention further provides a method for treatment of Ménière's disease, characterized by bringing the aforementioned Ménière's-disease-specific antigen into contact with biological components of a patient suffering from Ménière's disease, to thereby remove an antibody unique to Ménière's disease from the biological components, and subsequently, returning the biological components into the patient's body.

### BRIEF DESCRIPTION OF THE DRAWINGS

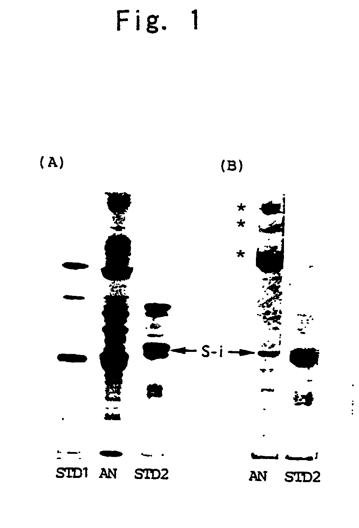

Fig. 1 is a photograph showing the results of TLC immunostaining of a human acoustic-tumor ganglioside by use of serum of a patient of Ménière's disease, in which Fig. 1(A) shows the results of resorcinol staining, and Fig. 1(B) shows the results of immunostaining. The stained bands marked with * are non-specific bands.

### BEST MODE FOR CARRYING OUT THE INVENTION

The present invention is drawn to a ganglioside regarded as a likely onset factor of Ménière's disease and having a specific reactivity with an antibody unique to the disease. The ganglioside has the following sequence: NeuAcα2-3Galβ1-4GlcNAcβ1-3Galβ1-4GlcNAcβ1-3Galβ1-4Glcβ1-1Cer, and has fundamental characteristics based on the sequence. A ganglioside having a small modification in the above sequence is also encompassed by the ganglioside of the present invention so long as the ganglioside has reactivity with an antibody unique to Ménière's disease.

As used herein, the antibody unique to Ménière's disease refers to an autoantibody or an immune complex which acts as an onset factor of symptoms of Ménière's disease, and is an antibody specifically contained in biological components of a patient suffering from the disease.

The expression "has specific reactivity with an antibody unique to Ménière's disease" refers to that the ganglioside of the present invention reacts with an antibody unique to Ménière's disease (an autoantibody or an immune complex) which can be detected by use of a known detection method; for example, the detection method described herein, to thereby form an antigen-antibody complex. In this sense, the antibody unique to Ménière's disease referred to herein can be described as being an antiganglioside antibody.

In the present invention, the biological components are not limited, so long as they contain the above-described antibodies. Examples of the biological components include blood constituents such as plasma or serum, spinal fluid, lymph, urine, tear, and milk. Preferably, the biological components are blood constituents such as plasma or serum.

Examples of subjects from which the biological components of the present invention are obtained include vertebrates; particularly mammals including domestic animals, primates, and *Homo sapiens*. Preferably, the subject is *Homo sapiens*.

The above-described specific ganglioside of the present invention is known as i-type ganglioside, and can be prepared according to a customary method (Taki T, *et al*. (1988) Lipids 23, 192.).

The present invention provides a useful material for analysis of a biological sample regarding the presence of an antibody unique to Ménière's disease, as well as a reagent kit comprising the material. That is, the present invention provides an antigen having a specific reactivity with an antibody unique to Ménière's disease to be detected; more specifically, a material including an antigen comprising the above-described ganglioside, and a reagent kit comprising the material.

The above-described ganglioside can be, if desired, fixed into a solid-phase antigen by way of immobilization onto a carrier, and the obtained antigen is very useful, particularly as a selective absorbent of an antibody unique to Ménière's disease.

In the present specification, the carrier is not limited to a specific material, so long as the carrier is a non-soluble, inactive carrier. A variety of commonly-used carriers can be used. Examples of the carrier include a stick, beads, micro-plates, a test tube, film, and powder, and they may be made of a variety of materials such as glass, cellulose, agarose gel, polystyrene, filter paper, carboxymethyl cellulose, ion-exchange resin, dextran, plastic film, plastic tube, nylon, silk, polyamine-methyl vinyl ether-maleic acid copolymer, amino acid copolymer, and ethylene-maleic acid copolymer.

The immobilization method is not limited, and both physical bonding and chemical bonding methods can be used. Examples of covalent bonding methods include chemical-reaction methods such as a diazo method; a peptide method (an acid-amide-derivative method, a carboxyl-chloride resin method, a carbodiimide-resin method, a maleic-anhydride-derivative method, an isocyanate-derivative method, a cyanogen-bromide-activated-polysaccharide method, a cellulose-carbonate-derivative method, or a method which makes use of a condensation reagent); an alkylation method; a carrier-bonding method making use of a crosslinking reagent (glutaraldehyde and hexamethylene isocyanate are used as the cross-linking reagent); and a carrier-bonding method making use of Ugi reaction; and examples of ion bonding methods include a method through use of a carrier, wherein an ion-exchange resin is used as the carrier. Examples of physical adsorption methods include a carrier-using method in which porous glass such as glass beads is used.

The Ménière's-disease-specific antigen of the present invention can absorb and capture an autoantibody serving as an onset factor of Ménière's disease, through immunological reaction with the autoantibody. Therefore, the antigen of the present invention is useful for detection of the presence of the autoantibody in a biological sample which may include blood or a serum sample. Further, the use of the antigen enables provision of the antibody unique to Ménière's disease as a material for analytical research by isolating the antibody from the biological components, and enables isolation and removal of an antibody unique to Ménière's disease from human plasma.

The present invention provides a reagent comprising such a Ménière's-disease-specific antigen, as well as a reagent kit for detecting an antibody unique to Ménière's disease.

The reagent kit of the present invention is characterized by including, as an active component, the above-described Ménière's-disease-specific antigen; however, the kit may be a combination of the antibody and at least one to four members optionally selected from the group consisting of a solid phase, a labeling agent, a substrate suitable for the labeling agent (a detection reagent), and an antihuman immunoglobulin antibody serving as a secondary antibody.

In this regard, when the set includes a labeling agent, the optional component such as a secondary antibody may have been conjugated with the labeling agent in advance. Examples of the labeling agent include a variety of compounds such as a radioisotope, an enzyme, and a fluorescent substance, wherein an enzyme is preferable from the viewpoint of ease of use.

Further, from the viewpoint of convenience for measurement, the reagent kit may contain a suitable antibody diluting solution, a reaction diluting solution, a standard antibody, a buffer, a washing solution, a substrate solution, and a reaction stopper solution. The antibody reagent may be freeze-dried by addition of thimerosal or glycerine.

Further, the present invention covers an assay for detection of the antibody unique to Ménière's disease contained in biological components.

The assay includes a variety of methods used in the art for detection of an antibody or an immune complex contained in biological components. Examples of the assay include agar gel immunodiffusion, agglutination reaction, agglutination inhibition reaction, counter-immunoelectrophoresis, immunofluorescence microscopy, an immunoelectron microscopy, radioimmunoassay, and an enzyme-linked immunosorbent assay.

Preferably, the assay is an immunoassay which includes, as essential steps, an immunoassay comprising: a step in which a biological sample of a patient suspected of suffering from Ménière's disease is incubated together with an antigen for an autoantibody to be detected (the Ménière's-disease-specific antigen of the present invention) under the conditions under which an antigen-antibody complex is produced; and a step in which the produced antigen-antibody complex is detected. Specifically, the assay includes an immunoassay, such as a competitive assay or a sandwich assay.

More particularly, when a solid phase sandwich assay which employs human serum as a test sample is taken as an example, the target antibody unique to Ménière's disease can be measured by the following method, for example. First, the Ménière's-disease-specific antigen of the present invention which specifically reacts with the antibody to be assayed is immobilized onto a solid phase, and a serum specimen which serves as the test sample is added thereto. As a result, an antigen-antibody reaction occurs between the immobilized antigen and the autoantibody present in the test sample, and the target antibody present in the specimen binds to the immobilized antigen. Subsequently, through detection of the amount of the bound antigen by use of a human antibody detective reagent containing a labeled antihuman immunoglobulin antibody, the antibody unique to Ménière's disease present in the test sample can be detected and measured.

In the above-described process, the target autoantibody present in the test sample can also be detected and measured by the following procedure: the human antibody detective reagent is immobilized onto a solid phase for capturing the antibody present in the specimen; the antigen of the present invention is subsequently added thereto, to thereby bind the antigen to the antibody unique to Ménière's disease; and the labeled antibody specific to the antigen is bound thereto. Selection and modification of a variety of means in these assay methods are well known by persons skilled in the art, and any one of such methods can be used in the present invention (Clinical Examination Handbook, Kinbara Press Co., 1995). The designs of the immunoassay may involve numerous alterations usually carried out in the art.

The labeling agent used in each assay format is not limited, and both conventionally known agents and agents to be used in the near future may be used. Specific examples of the labeling agent include a radioisotope such as ³H or ¹⁴C, an enzyme such as alkaline phosphatase or peroxidase (POX), a fluorescent substance such as fluorescein isothiocyanate (FITC) or tetramethylrhodamine isothiocyanate (RITC), and 1N-(2,2,6,6-tetramethyl-1-oxyl-4-piperidyl)-5N-(aspartate)-2,4-dinitrobenzene (TOPA). The immunoassays using these labeling agents are called radioimmunoassay, enzyme immunoassay, fluoroimmunoassay, and spin immunoassay, respectively. In the present invention, enzyme immunoassay using an enzyme as a labeling agent is preferably performed from the viewpoints of simplicity, safety, and sensitivity.

Examples of the substance for labeling enzyme include, in addition to the above-described substances, microperoxidase, chymotrypsinogen, procarboxypeptidase, glyceroaldehyde-3-phosphoric acid dehydrating enzyme, amylase, phosphorylase, D-nase, and P-nase. The labeling methods making use of these labeling substances can be performed according to a method known *per se* ("Monoclonal Antibody" by Tatsuo Iwasaki, *et al*., Kodansha Scientific, 1984; "Enzyme Immunoassay" second edition, by Eiji Ishikawa, *et al*., Igaku Shoin, 1982).

The activity of enzyme may be measured by use of a known method in accordance with the type of the enzyme employed. For example, when peroxidase is used as a labeling enzyme, the substrate may be ABTSJ 2,2'-azino-bi(3'-ethylbenzthiazoline sulfonate), and when alkaline phosphatase is used as a labeling enzyme, the substrate may be p-nitrophenyl phosphate; incubation is performed; and then decomposition of the substrate is measured by use of a spectrophotometer or a similar device ("Enzyme Immunoassay" second edition, by Eiji Ishikawa, *at al*., Igaku Shoin, 1982).

Alternatively, assays making use of biotin and avidin may be performed, or assays amplifying signals from probes such as an enzyme label immunoassay or an enzyme-mediated immunoassay (for example, ELISA) may be used.

In the case in which a substance labeled with a radioisotope or a fluorophor is used instead of the above-described enzyme, measurement may be carried out according to a method known *per se*.

A customarily used solvent may be used as a solvent for the above-described measuring system, so long as the solvent does not exert an undesirable influence upon the reaction. Examples of the solvent include a buffer solution having a pH of about 5-9, such as a citrate buffer solution, a phosphate buffer solution, a Tris salt buffer solution, or an acetate buffer solution.

Conditions for immunological reaction (bonding) are not limited, and conditions customarily used in assays of the above-described types may be used. The reaction may be performed under conditions in which the temperature is 45°C or less, preferably about 4-40°C, and the reaction time is about 1-40 hours.

When the above-described method is carried out, use of the above-described immunoassay reagent kit of the present invention is convenient.

The present invention provides a reagent kit comprising a Ménière's-disease-specific antigen of the present invention, which kit is useful for selective isolation or removal of the antibody unique to Ménière's disease.

In the treatment of patients suffering from a variety of autoimmune diseases, a therapeutic method such as hemocatharsis or plasmapheresis has conventionally been used so as to remove from the patient an autoantigen or an immune complex which is known to be specific to the symptoms or the cause of the diseases. Further, methods for specifically isolating or removing an antibody of interest by use of an antigen corresponding to the antibody have also been known (see, for example, Taki T, *et al*. (1981) J. Biochem. 89, 503-510; Taki T, *at el*. (1982) J. Biochem. 91, 1813-1816; Hirabayashi Y, *et al*. (1983) J. Biochem. 94, 327-330; Yuki N, *et al*. (1996) Neurology 46, 1644-1651). These methods may be used in the same way in which hemocatharsis or plasmapheresis is carried out.

The above-described reagent kit according to the present invention can be used in the same way in which isolation or removal is used, based upon the use of the specific antigen of the present invention. The reagent kit is used for selective and specific removal, from the biological components, of an autoantigen or an immune complex unique to Ménière's disease. Thus, the present invention provides a new therapy for Ménière's disease enabling removal of the specific antibody, as a substitute for the conventional plasmapheresis. Therefore, the reagent kit of the present invention may incorporate a variety of reagents, drugs, and devices that are used in plasmapheresis. Preferably, in the reagent kit of the present invention, the Ménière's-disease-specific antigen of the present invention may be an absorbent comprising a carrier onto which the above-described ganglioside has been immobilized. Such carrier is not limited, so long as it is generally used for plasmapheresis. Examples of the carrier include the above-described different carriers, such as a variety of beads including magnetic beads, film, and plastic film.

Moreover, the present invention is also directed to a method for isolating and removing an antibody unique to Ménière's disease, characterized in that an antigen specific to Ménière's disease of the present invention is brought into contact with a sample containing the antibody unique to Ménière's disease. The use of this method enables specific isolation, from biological components, of an autoantibody or an immune complex regarded as an onset factor of Ménière's disease. Therefore, use of the method can contribute to the basic study toward the clarification of the structure of the antibody, the physiological significance of the structure, and the onset mechanism of Ménière's disease.

Furthermore, a detective reagent which can specifically detect the antibody unique to Ménière's disease or a removing material which can selectively isolate or remove the antibody from biological components can be produced by use of immunoglobulin produced from antiserum obtained from a mammal immunized by use of the antibody isolated by the above-described method, or by use of a monoclonal antibody obtained from hybridomas produced from antibody-producing cells and myeloma cells obtained from the mammal.

### Examples

The present invention will next be described in detail by way of examples, which should not be construed as limiting the invention thereto.

### (1) Separation of a ganglioside fraction from acoustic neurinoma

Ganglioside was separated by way of extraction from acoustic neurinoma. Specifically, lipid was extracted from neurinoma extirpated from a patient, by use of chloroform : methanol (2:1, v/v). The obtained lipid was subjected to separation by use of an anion exchange column (DEAE-Sephadex), to thereby separate into an acidic fraction and a neutral fraction. Subsequently, the acidic fraction was dialyzed by use of a Sep-Pak C₁₈ column, to thereby obtain a fraction containing ganglioside.

Thereafter, the resultant fraction was subjected to high performance thin layer chromatography (HPTLC), developed by use of chloroform/methanol/0.2% CaCl₂ (55/45/10), and stained by use of resorcinol (Seriwanov Reagent Chemicals). Ganglioside obtained from seven cases of acoustic neurinoma exhibited almost the same patterns. In addition to a major band corresponding to GDla, there were detected 6-7 other bands which were resorcinol-positive and had polarity lower than the primary band.

All the bands were subjected to structure analysis by use of an analysis method which was a combination of TLC blocking/MS and TLC immunostaining.

That is, the ganglioside developed under UV light by HPTLC was colored by use of a primuline reagent, marked with a colored pencil, and transferred to a PVDF film by TLC blotting [Taki T, *et al*. (1994) Anal. Biochem 223, 232]. The transferred PVDF film was directly subjected to mass spectroscopy by use of direct SIMS (secondary ion mass spectrometry) according to the method reported in the literature [Taki T, *et al*. (1995) Anal. Biochem 225, 24], to thereby perform structure analysis.

Thus, eight gangliosides; that is, GM3, GM2, SPG, GMla, Hex-GMl (or Hex-SPG), GD3, i-type ganglioside, and GDla, were identified. The peripheral nervous system was reported to have a great deal of SPG (Svennerholm L, *et al.* (1994) Biochim. Biophys. Acta 1214. 115.). However. GDla was dominant in all seven cases of acoustic neurinoma.

### (2) TLC immunostaining

The above-described gangliosides extracted from acoustic neurinoma and developed by TLC were used as antigens, and reacted overnight with serum (25-fold diluted) from 11 cases diagnosed as Ménière's disease, followed by a further reaction with a second antibody, i.e., peroxidase-labeled antihuman γ chain or µ chain specific antibody (1000-fold diluted) for 2 hours and subsequent color development by use of Konica Immunostain (manufactured by Konica).

The results are shown in Fig. 1. In Fig. 1, (A) shows the results of the above-described resorcinol staining, and (B) shows the results of the above-described immunostaining. The lanes are as follows.
- STDl:: Standard gangliosides including GM3, GM2, and BBG (bovine brain gangliosides).
- AN:: Acoustic neurinoma ganglioside.
- STD2:: Standard ganglioside including SPG, S-i (i-type ganglioside, and S-I (I-type ganglioside).

In Fig. 1, the band corresponding to i-type ganglioside was found to provide positive results (i.e., immunostained). Further, MS analysis of this band confirmed that the band was in fact in agreement with the i-type ganglioside.

In the above-described immunostaining, serum samples from 5 cases out of 11 cases provided positive reaction with the i-type ganglioside, and one reference out of 8 normal control cases showed positive reaction.

On the basis of the above-described results, i-type ganglioside is considered to be an antigen specific to Ménière's disease.

## Claims

1. A Ménière's-disease-specific antigen comprising a ganglioside having the following sequence: NeuAcα2-3Galβ1-4GlcNAcβ1-3Galβ1-4GlcNAcβ1-3Galβ1-4Glcβ1-1Cer.

2. The specific antigen according to claim 1, wherein the ganglioside is immobilized onto a carrier.

3. A reagent kit for detecting an antibody unique to Ménière's disease, characterized by comprising the specific antigen as described in claim 1.

4. An immunoassay for detecting an antibody unique to Ménière's disease, characterized by causing the specific antigen described in claim 1 to react with biological components and detecting a produced antigen-antibody complex comprising the specific antigen.

5. The immunoassay according to claim 4, wherein the step for detecting the antigen-antibody complex comprising the specific antigen comprises incubation of the immunocomplex with an enzyme-labeled or radioisotope-labeled anti-human immunoglobulin antibody.

6. A reagent kit for selective isolation or removal of an antibody unique to Ménière's disease, comprising the specific antigen described in claim 1.

7. A method for isolating or removing an antibody unique to Ménière's disease, characterized by bringing the specific antigen described in claim 1 into contact with biological components containing an antibody unique to the disease.

8. A diagnostic method for Ménière's disease, characterized by causing the specific antigen described in claim 1 to react with biological components and detecting a produced antigen-antibody complex comprising the specific antigen.

9. The diagnostic method according to claim 8, wherein the step for detecting the antigen-antibody complex comprising the specific antigen comprises incubation of the immunocomplex with an enzyme-labeled or radioisotope-labeled anti-human immunoglobulin antibody.

10. A method for treatment of Ménière's disease, characterized by bringing the specific antigen described in claim 1 into contact with biological components of a patient suffering from Ménière's disease, to thereby remove an antibody unique to Ménière's disease from the biological components, and subsequently, returning the biological components into the patient's body.
